(19) Europäisches Patentamt
European Patent Office
Office européen des brevets

(11) **EP 2 540 388 B1**

(12) **EUROPÄISCHE PATENTSCHRIFT**

(45) Veröffentlichungstag und Bekanntmachung des Hinweises auf die Patenterteilung:
**08.03.2017 Patentblatt 2017/10**

(51) Int Cl.:
**B01J 8/08** *(2006.01)* **C07C 1/12** *(2006.01)*
**C10L 3/08** *(2006.01)* **B01J 8/02** *(2006.01)*
**C07C 29/152** *(2006.01)*

(21) Anmeldenummer: **12004865.7**

(22) Anmeldetag: **29.06.2012**

(54) **Verfahren zum Betreiben eines Methanisierungsreaktors**

Method for operating a methanation reactor

Procédé destiné au fonctionnement d'un réacteur de méthanisation

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.06.2011 DE 102011105934**

(43) Veröffentlichungstag der Anmeldung:
**02.01.2013 Patentblatt 2013/01**

(73) Patentinhaber: **ETOGAS GmbH**
**70565 Stuttgart (DE)**

(72) Erfinder:
• **Buxbaum, Martin**
**6861 Alberschwende (AT)**

• **Waldstein, Gregor**
**70565 Stuttgart (DE)**

(74) Vertreter: **Leinweber & Zimmermann**
**European Patent Attorneys**
**Patentanwälte**
**Rosental 7**
**80331 München (DE)**

(56) Entgegenhaltungen:
**US-A1- 2006 231 500    US-A1- 2007 142 204**
**US-A1- 2010 162 627    US-A1- 2011 062 722**
**US-B1- 7 757 541**

**Beschreibung**

Die Erfindung betrifft ein Verfahren zum Betreiben eines Methanisierungsreaktors, bei dem in einer ersten Betriebsart ein Kohlendioxid und Wasserstoff aufweisendes Eduktgas entlang eines Strömungsweges des Reaktors mit einem ersten Stoffumsatz katalytisch zu einem methanreichen Produktgas methanisiert wird.

Derartige Methanisierungsverfahren auf katalytischer Basis sind seit über 100 Jahren bekannt. Sie werden in Reaktorsystemen realisiert, welche in der Regel fortlaufend mit gleichbleibender, konstant hoher Auslastung gefahren werden. Selbstverständlich muß dabei, wenn auch so selten wie möglich, das Reaktorsystem stillgelegt werden, um Wartungsarbeiten durchführen zu können, oder auch, um die Katalysatoren des Reaktorsystems zu ersetzen. Üblicherweise leidet der Katalysator unter Auslastungswechseln und wird nach ca. 1 bis 5 Abstellungen ausgetauscht.

Der Erfindung liegt die Aufgabe zugrunde, ein Verfahren der eingangs genannten Art unter geringer Belastung für den Katalysator flexibler zu gestalten.

In verfahrenstechnischer Hinsicht wird diese Aufgabe im wesentlichen dadurch gelöst, daß man wenigstens am Beginn des Strömungsweges in einer zweiten Betriebsart mit geringerem oder verschwindendem zweiten Stoffumsatz einen methanreichen Gasstrom strömen läßt. Auf diese Weise wird nicht nur der Katalysator des Reaktors geschont, indem man sich dem Idealzustand annähert, gemäß dem eine Katalysatorbelastung nur bei Stoffumsatz, d.h. bei Last auftritt. Es wird auch die Grundlage dafür geschaffen, daß der Reaktor von der zweiten Betriebsart (z.B. Stoffumsatz im wesentlichen gleich Null) rasch wieder in die erste Betriebsart geschaltet werden kann.

Unter dem Ausdruck "methanreiches Produktgas" ist ein Gasgemisch zu verstehen, dessen Methangehalt 84 % oder mehr, bevorzugt 88 % oder mehr, insbesondere 92 % oder mehr beträgt. Der maximale $CH_4$-Gehalt des Produktgases ist dabei von der Gleichgewichtsbedingung der folgenden, bei der Methanisierung des Eduktgases im wesentlichen stattfindenden und in Summe $CH_4$ bildenden Reaktionen

1) $CO + H_2O \leftrightarrow CO_2 + H_2$, die sogenannte watergas shift reaction
2) $CO + 3\,H_2 \; CH_4 + H_2O$, die CO-Methanisierung und
3) $CO_2 + 4\,H_2 \leftrightarrow CH_4 + 2\,H_2O$, die $CO_2$-Methanisierung limitiert.

Es treten des Weiteren Reaktionen auf, bei denen in geringem Maße elementarer Kohlenstoff erzeugt wird, der sich auch katalysatorschädlich ablagern kann. Diesen Ablagerungen kann durch $H_2O$-Zugabe entgegengewirkt werden.

Für den Methananteil des methanreichen Gasstroms wird es als ausreichend angesehen, wenn dieser wenigstens 30 %, bevorzugt wenigstens 50 %, insbesondere wenigstens 78 % beträgt und im übrigen insbesondere an den Methangehalt des Produktgases heranreicht, bezogen auf die Gasmenge ohne $H_2O$-Anteil.

Thermodynamische Einstellungen wie z.B. der Druck im Reaktor können in den Betriebsarten zusätzlich geändert werden, aber auch gleich bleiben. Die Betriebsarten definieren sich durch den Stoffumsatz im Reaktor (auch: die Last). Dadurch kann berücksichtigt werden, daß rasche Druck- und/oder Temperaturwechsel, also große Temperaturunterschiede im Reaktor vermieden werden, auf die der Katalysator empfindlich ist.

In einer besonders bevorzugten Verfahrensgestaltung zirkuliert der methanreiche Gasstrom auf einem Zirkulationsweg, der den Strömungsweg wenigstens teilweise, insbesondere vollständig enthält. Auf diese Weise geht der hier als schützender Gasstrom verwendete methanreiche Gasstrom nicht verloren, sondern kann später wiederum zu ausgekoppeltem Produktgas werden.

Für eine bevorzugte Verfahrensgestaltung kann der methanreiche Gasstrom einen Methangehalt aufweisen wie das in der ersten Betriebsart erzeugte Produktgas. Während die erste Betriebsart einer Auslastung des Reaktors bei vorgegebenem Auslastungsgrad, bevorzugt Vollauslastung, entspricht, ist für die zweite Betriebsart bevorzugt der Reaktorstillstand vorgesehen, bei dem dem Strömungsweg kein Eduktgas mehr zugeführt wird. Auf diese Weise umfaßt die erreichbare Flexibilität des Verfahrens auch noch den stoffumsatzmäßig gesehenen Reaktorstillstand. Ein vollständiges Herunterfahren des Reaktors im Sinne einer Druckentspannung und eines Auskühlens ist bei einem solchen Lastwechsel nicht erforderlich.

Entsprechend wird bei Reaktorstillstand auch kein Produktgas mehr erzeugt wie in der ersten Betriebsart, in der das Produktgas aus einer insbesondere an dem Ende des gemeinsamen Weges von Strömungsweg oder dessen Anschlußweg und Zirkulationsweg gebildeten Weiche ausgekoppelt wird. Demnach wird bei Stillstand auch kein Produktgas mehr aus dem Reaktor ausgekoppelt, die Weiche ist auf 100 % Rezirkulation eingestellt.

In einer besonders bevorzugten Ausführungsform wird mit dem zirkulierenden methanreichen Gasstrom ein Wärmeausgleich zwischen unterschiedlichen Orten entlang des Strömungsweges und/oder Zirkulationsweges bewirkt. Auf diese Weise kann das gesamte Reaktorsystem durch Wärmezufuhr an nur einer Wärmequelle auf einer insbesondere für ein schnelles Hochfahren des Reaktors erforderlichen Temperatur gehalten werden.

Dabei ist es nicht zwingend erforderlich, Parameter wie z.B. Strömungsgeschwindigkeiten über die gesamte Dauer des Betriebs in der zweiten Betriebsart aufrechtzuerhalten. Beispielsweise ist es für den Wärmeausgleich ausreichend, 70 % oder weniger, bevorzugt 50 % oder weniger, insbesondere 30 % oder weniger der Strömungsgeschwindigkeit zu halten, welche auf dem Strömungsweg in der ersten Betriebsart herrscht.

Besonders zweckmäßig wird der Zirkulationsweg auch in der ersten Betriebsart verwendet, und zwar zur anteiligen Rezirkulation des Produktgases. Auf diese Weise kann die Reaktionsgeschwindigkeit insbesondere am bzw. kurz hinter dem Reaktoreingang geeignet herabgesetzt werden, um der Entstehung der sogenannten Hot Spots entgegenzuwirken. Das Verhältnis des Volumenstroms des rezirkulierten Gases zu dem des Eduktgases/Produktgases kann dabei in der ersten Betriebsart durchaus auch größer als 1 sein und insbesondere auch höher, z.B. bis zu 5 gewählt werden, es kann jedoch auch kleiner als 1 sein.

Besonders bevorzugt werden beim Übergang von dem ersten zum zweiten Betriebszustand aufeinander abgestimmt der Eduktgasstrom heruntergefahren und der Anteil des ausgekoppelten Produktgases heruntergefahren, und vice versa. Diese Abstimmung wird insbesondere in Abhängigkeit davon gesteuert, welcher absolute Volumenstrom für den zirkulierenden Gasstrom angestrebt wird. Beim Übergang vom zweiten in den ersten Betriebszustand kann insbesondere die Erhöhung des Anteils des ausgekoppelten Produktgases auch zeitlich verzögert erfolgen, um den Methangehalt des ausgekoppelten Gasstroms konstant hoch zu halten.

Grundsätzlich kann der methanreiche Gasstrom für die Dauer bis zum erneuten Übergang zur ersten Betriebsart aufrechterhalten werden. Sofern ein längerer Stillstand (d.h. Stoffumsatz effektiv Null, aber kein Herunterfahren im Sinne von Druckentspannung und Auskühlung) geplant oder erwartet wird, kann der methanreiche Gasstrom grundsätzlich auf Strömungsgeschwindigkeit 0, also mit Wirkung lediglich einer Reaktorflutung gesenkt werden, und gegebenenfalls auch die Wärmezufuhr über eine Wärmequelle abgesenkt werden. Eine entsprechende Wiederherstellung der Strömungsgeschwindigkeit und Wärmezufuhr kann z.B. nach Erhalt eines Vorwarnsignals wieder aufgenommen werden, mit dem ein Hochfahren des Reaktors nach einer vorgegebenen Vorwarnzeit angekündigt wird. Es wird aber bevorzugt, wenn die Temperatur nicht tiefer als die Anspringtemperatur des Katalysators, insbesondere nicht tiefer als ca. 290° C abgesenkt wird.

Besonders bevorzugt wird der Reaktor zwischen der ersten und der zweiten Betriebsart abhängig von der für die insbesondere raumnahe Erzeugung des Wasserstoffes für das Eduktgas bereitgestellten elektrischen Leistung intermittierend betrieben. Auf diese Weise kann der Reaktor eingesetzt werden, wenn elektrische Leistung günstig bereitgestellt werden kann, und anderweitig auf Stand-by gelegt werden, wobei der entsprechende Lastwechsel des Reaktors schonend erfolgt. Aufgrund des erfindungsgemäß günstigen Stand-by-Betriebes des Reaktors kann dieser ohne Beschädigungen auch wenigstens 10 Mal pro Monat, bevorzugt wenigstens 5 Mal pro Woche, insbesondere wenigstens 2 Mal pro Tag zwischen den Betriebsarten umgeschaltet werden.

Während der Reaktor in der zweiten Betriebsart durchschnittlich wenigstens 10 Minuten oder mehr, auch 30 Minuten oder mehr, insbesondere auch 1 Stunde oder mehr gefahren werden kann, können geringe Umschaltzeiten zwischen der ersten und der zweiten Betriebsart und/oder vice versa von 5 Minuten oder weniger, bevorzugt 3 Minuten oder weniger, insbesondere 2 Minuten oder weniger erreicht werden. Dadurch wird wertvolle, zur energetisch günstigen Methanherstellung nutzbare Zeit gespart.

Zur Vermeidung von schädlichen Kohlenstoffablagerungen am Katalysator wird dem methanreichen Gasstrom $H_2O$ zugeführt, insbesondere als Dampf. Der $H_2O$-Gehalt des zirkulierenden Stroms wird hierzu zweckmäßig überwacht und nach gewünschten Vorgaben geregelt. Des Weiteren kann dem zirkulierenden Gasstrom $H_2$ zugeführt werden, um einem etwaigen Oxidieren des Katalysators entgegenzuwirken.

Die Erfindung kann mit einem Methanisierungsreaktor umgesetzt werden, der neben den zur Durchführung des Verfahrens erforderlichen körperlichen Merkmalen wie Katalysatoren etc. eine Steuereinrichtung aufweist, die zur Steuerung des Reaktors nach dem erfindungsgemäßen Verfahren programmiert ist. Die Vorteile der erfindungsgemäßen Anlage ergeben sich aus der obigen Beschreibung des erfindungsgemäßen Verfahrens.

Weitere Merkmale, Einzelheiten und Vorteile der Erfindung ergeben sich aus der Beschreibung der beiliegenden Zeichnungen, von denen

Figur 1    eine schematische Darstellung des erfindungsgemäßen Verfahrens zeigt, und

Figur 2    ein qualitatives zeitliches Ablaufdiagramm zeigt.

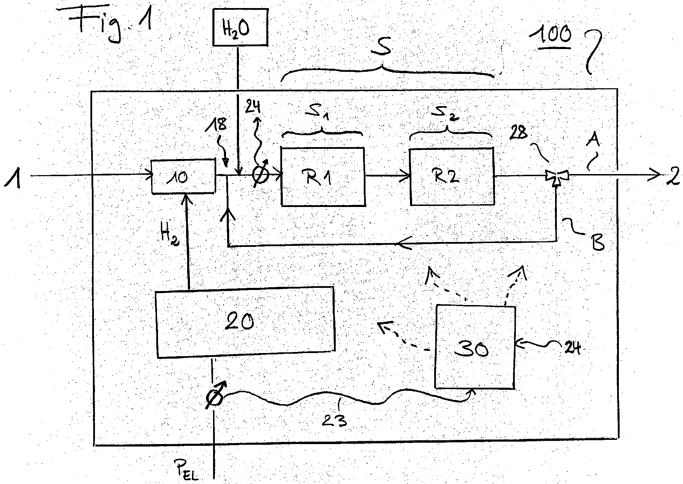

Figur 1 zeigt schematisch eine Baueinheit 100, welche einen Elektrolyseur 20 und einen in diesem Ausführungsbeispiel zweistufigen Methanisierungsreaktor R aufweist. Die Baueinheit 100 ist in der Lage, von einer Steuerungseinrichtung 30 gesteuert aus einem Kohlendioxid enthaltenden zugeführten Gas 1 und zugeführter elektrischer Leistung $P_{EL}$ ein methanreiches Produktgas 2 zu erzeugen. Dazu wird dem Kohlendioxid enthaltenden Gasgemisch 1, das bevorzugt bis auf Restbestandteile geringer Konzentration reines Kohlendioxid ist und ursprünglich z.B. aus Industrieabgasen oder auch Biogasanlagen stammen kann, aber auch ein neben Kohlendioxid noch einen Methananteil aufweisendes Gas sein kann, in einer Mischeinrichtung 10 Wasserstoff zugemischt, welcher von dem mit der elektrischen Leistung $P_{EL}$ gespeisten Elektrolyseur 20 erzeugt wird.

Nach Durchlauf des Strömungsweges S, der in diesem Ausführungsbeispiel des zweistufigen Reaktors R mit den Reaktorstufen R1 und R2 zwei Teilabschnitte S1 und S2 aufweist, entlang derer die Methanisierungsreaktion des aus dem zugeführten Gas 1 und dem zugemischten Wasserstoff gebildeten Eduktgases stattfindet, erhält man ein methanreiches Produktgas 2 mit hier etwa 92 % Methananteil (nach einer nicht dargestellten Wasserabscheidung aus dem Gas).

Im folgenden wird beschrieben, wie die Steuereinrichtung 30 die einzelnen Teile der Baueinheit 100 steuert, wie durch die gestrichelten Pfeile angedeutet ist.

In einem ersten Betriebszustand, der hier der Volllastbetrieb ist, wird in dem Reaktor R die maximale Eduktgasmenge umgeschlagen, für die der Reaktor R ausgelegt ist. Der für diesen maximalen Eduktgasstrom ausgelegte Elektrolyseur 20 stellt die für das stöchiometrische Verhältnis der Methanisierungsreaktion erforderliche Wasserstoffmenge bereit, gegebenenfalls etwas mehr oder weniger, je nach einer etwaig gewünschten Verschiebung des Gleichgewichts der Methanisierungsreaktion. Die dazu erforderliche elektrische Leistung, $P_{ELmax}$, wird dem Elektrolyseur 20 zur Verfügung gestellt, bevorzugt aus regenerativen Energiequellen wie z.B. Windenergie. Dabei kann die elektrische Leistung $P_{EL}$ unmittelbar von der Energiequelle bezogen werden oder indirekt über eine Einspeisung und Entnahme aus dem bestehenden Stromnetz.

Das auf diese Weise in einem maximalen Volumenstrom erzeugte methanreiche Produktgas kann entweder vor Ort genutzt, abgefüllt, anderweitig gespeichert oder auch nach entsprechender Konditionierung in ein bestehendes Gasnetz eingespeist werden.

Im Gegensatz zu üblichen Methanisierungsreaktoren, die in diesem Volllastbetrieb im wesentlichen durchgehend betrieben werden und nur zu Wartungszwecken oder Austausch der Katalysatoren des Reaktors ihren Betrieb unterbrechen, ist die Baueinheit 100 und der Reaktor R dazu ausgelegt, im intermittierenden Betrieb je nach schwankend bereitgestellter elektrischer Leistung $P_{EL}$ mit unterschiedlichen Umwandlungsraten (Stoffumsätzen) der Methanisierungsreaktion im Reaktor R gesteuert zu werden.

Die Steuereinrichtung 30 erhält dazu ein Signal 23, dessen Informationsgehalt die Höhe der bereitgestellten elektrischen Leistung wiedergibt. In Abhängigkeit von diesem Signal 23 wird der Zustrom des Kohlendioxid enthaltenden Gases 1 und die Menge des in den Reaktor R eingeleiteten Eduktgases gesteuert und insbesondere automatisch auf geringere Umwandlungsraten bis zum Stillstand der Baueinheit 100 einstellt. Die Zufuhr der Eduktgasbestandteile kann unabhängig voneinander gesteuert werden. Beispielsweise kann beim Übergang von der ersten zur zweiten Betriebsart der Kohlendioxidzustrom vor dem $H_2$-Zustrom abgestellt werden.

Im folgenden wird das Beispiel eines zweiten Betriebszustandes der Baueinheit 100 beschrieben, in dem dem Elektrolyseur 20 keine elektrische Leistung $P_{EL}$ mehr zugeführt wird und die Methanisierungsreaktion zum Erliegen kommt (die zweite Umwandlungsrate ist effektiv Null, es wird kein Produktgas 2 mehr erzeugt).

Bei der Umstellung von der ersten Betriebsart auf diese zweite Betriebsart wird passend zu der geringeren Zuführung an Eduktgas ein kontinuierlich oder diskontinuierlich wachsender Anteil entlang des Strömungsweges S strömenden Gases an der Weiche 28 von der Fortsetzung des Strömungsweges S in einen Rezirkulationsweg B geleitet, der an der in der Figur mit 18 bezeichneten Stelle wieder in die Zuleitung des Eduktgases einmündet, bevor der Strömungsweg S beginnt. Auf diese Weise verebbt der über die Leitung A abgeführte Produktgasstrom innerhalb einer vorgegebenen Zeitspanne T, während der Nicht-Methananteil des auf dem Strömungsweg S strömenden Gasstromes bereits am Beginn des Strömungsweges S immer geringer wird, bis dort ein Methangehalt erreicht wird, der dem des erzeugten Produktgases entspricht. Nach Abklingen der Methanisierungsreaktion im Reaktor entsteht dadurch ein zirkulierender methanreicher Gasstrom mit sehr hohem Methananteil von hier ebenfalls etwa 92 % und Reaktionskomponenten Kohlendioxid und Wasserstoff im Bereich des chemischen Gleichgewichtes der Methanisierungsreaktion.

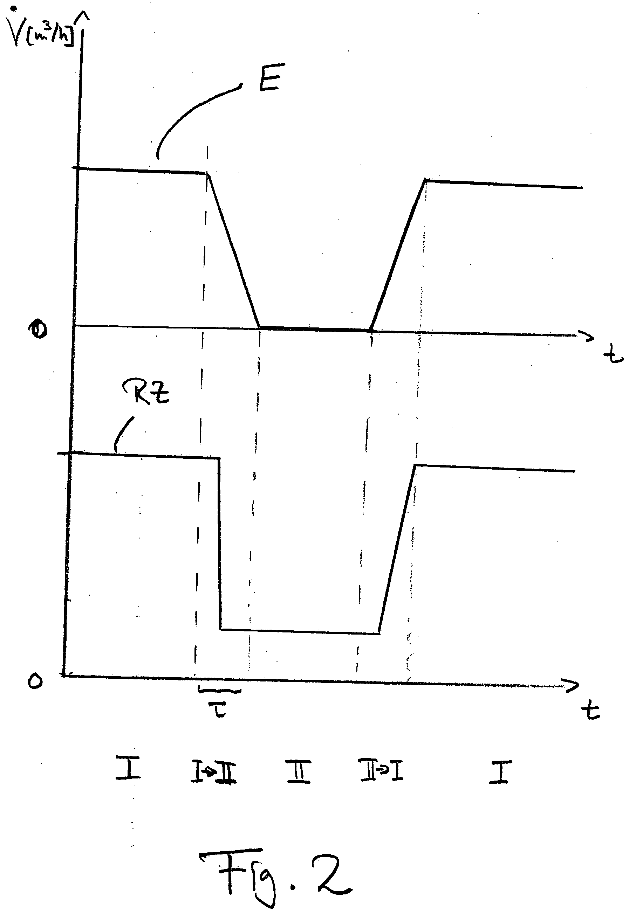

Diesen Lastwechsel von 100 % auf 0 % Auslastung zusammen mit einem Rückwechsel auf 100 % Auslastung sind in dem qualitativen Zeitdiagramm von Fig. 2 nochmals dargestellt. Darin steht RZ für den Volumenstrom des rezirkulierten Gases und E für den zugeführten Volumenstrom $\Delta v/\Delta t$ als Maß für die Auslastung des Reaktors, jeweils in $[m^3/h]$. Bezeichnet man diese Volumenströme mit $v'_{RZ}$ und $v'_{Edukt}$, so gilt in der zweiten Betriebsart

$$\arctan (v^`_{RZ} / v^`_{Edukt}) = \pi /2$$

Hinter der mit 18 bezeichneten Stelle, aber vor Reaktoreintritt wird über eine Regelung $H_2O$ zugegeben, um den Katalysator vor Kohlenstoffablagerungen zu schützen. Dazu wird der $H_2O$-Gehalt gemessen und mit Signal 24 an die Steuereinrichtung 30 signalisiert, welche nach vorgegebenen Kriterien einen Sollwert festgelegt hat und die Wasserzugabe auf diesen hin regelt. In ähnlicher Weise kann vor der Weiche 28 ein nicht dargestellter Wasserabscheider angeordnet sein und geregelt werden.

In einer alternativen Ausführungsform kann dem zirkulierenden Gasstrom noch zusätzlicher Wasserstoff zugeführt werden, um die Schutzwirkung für den Reaktor nochmals zu verbessern und insbesondere einer Oxidierungsgefahr des Reaktors entgegenzuwirken. Insbesondere kann in dieser abgewandelten Ausführungsform die vollständige Umschaltung der Weiche 28 abgeschlossen werden, bevor der Wasserstoffstrom des Elektrolyseurs 20 versiegt und im Gleichgang die Zufuhr des kohlendioxidreichen Gases 1 gestoppt wird.

Grundsätzlich könnte, sobald auf diese Weise eine geeigneter Schutz des Katalysators bewirkt wurde, auf eine weitere Einflußnahme auf den Reaktor verzichtet werden. Dann würde der zirkulierende methanreiche Gasstrom dissipativ zum Erliegen kommen. Bei dem hier dargestellten Ausführungsbeispiel wird jedoch über eine in der Zeichnung nicht dargestellte, in den Zirkulationsweg eingekoppelte Einrichtung eine Aufrechterhaltung des zirkulierenden methanreichen Gasstromes bewirkt. Dadurch wird eine auf einer Umwälzung beruhende Wärmeumverteilung innerhalb des Reaktorsystems erreicht, die dazu ausgenutzt werden kann, alle an den Zirkulationsweg wärmemäßig angekoppelten Teile durch Wärmezufuhr an nur einer Stelle auf einer gewünschten, über der Ansprintemperatur des Katalysators liegenden Temperatur, insbesondere über 290°C zu halten, welche einen raschen Übergang in die erste Betriebsart ermöglicht, sobald wieder elektrische Leistung zur Verfügung steht.

Für diesen inneren Wärmeausgleich ist es ausreichend, das methanreiche Gas mit einer vergleichsweise geringen Strömungsgeschwindigkeit strömen zu lassen, die insbesondere deutlich niedriger sein kann als die in der ersten Betriebsart auf dem Strömungsweg herrschenden Strömungsgeschwindigkeiten, beispielsweise nur noch 20 %.

Das Lasthochfahren des Reaktors R in die erste Betriebsart, d.h. z. B. auf Volllast, erfolgt prinzipiell durch ein umgekehrtes Schalten wie beim Lastabsenken des Reaktors. So wird die Zufuhr von Eduktgas kontinuierlich oder diskontinuierlich erhöht. Zeitlich angepaßt oder auch in einer solchen Art und Weise zeitlich versetzt, bis die Reaktion im Reaktor mit einer ausreichend hohen Reaktionsrate insbesondere nahe der ersten Umwandlungsrate in Gang gekommen ist, wird der auf der Rezirkulation B strömende Gasanteil verringert und der als Produktgas ausgekoppelte Gasanteil erhöht. Aufgrund der mit dem rezirkulierenden Methangasstrom gewährleisteten Hochfahrbereitschaft des Reaktors kann die Baueinheit 100 bereits ca. 2 Minuten nach Wiederherstellung der Zufuhr an elektrischer Leistung die Produktgasherstellung wieder aufnehmen. Dadurch qualifiziert sich die Baueinheit 100 für den intermittierenden Betrieb, und sie kann somit flexibel zur Ausnutzung von schwankend bereitgestellter elektrischer Leistung genutzt werden. Der Übergang von der zweiten auf die erste Betriebsart ist ebenfalls in Fig. 2 zu sehen.

Selbstverständlich ist diese Erfindung auch für Varianten einsetzbar, in denen der Reaktor R aus nur einer einzigen Reaktorstufe besteht, oder auch aus drei oder mehr Reaktorstufen. Des Weiteren kann, muß der Elektrolyseur 20 nicht zu einer gemeinsamen Baueinheit mit dem Reaktor R verbunden sein; es könnten auch vollkommen getrennte Anlagen zum Einsatz kommen.

Auch bei der ersten Betriebsart, hier Volllast, ist die Weiche 28 nicht zwangsläufig auf 100 % Produktgasauskopplung geschaltet, sondern kann insbesondere zur Temperaturregulierung über die Steuerung der Reaktionsgeschwindigkeit am Eingang des Reaktors zur Vermeidung der Bildung von Hot Spots auf eine anteilige Rezirkulation in geeigneter Höhe geschaltet werden, mit $v'_{RZ} / v'_{Edukt}$ im Bereich von einigen % bis hin zu Werten von z.B. 5.

Die Erfindung ist nicht auf das dargestellte Ausführungsbeispiel eingeschränkt. Vielmehr können die in den Ansprüchen und der Beschreibung offenbarten Merkmale einzeln oder in Kombination für die Verwirklichung der Erfindung in ihren verschiedenen Ausführungsformen wesentlich sein.

**Patentansprüche**

1. Verfahren zum Betreiben eines Methanisierungsreaktors (R), bei dem in einer ersten Betriebsart ein Kohlendioxid und Wasserstoff aufweisendes Eduktgas entlang eines Strömungsweges (S) des Reaktors (R) mit einer ersten Umwandlungsrate katalytisch zu einem methanreichen Produktgas (2) methanisiert wird,
   **dadurch gekennzeichnet, daß**
   man wenigstens am Beginn des Strömungsweges in einer zweiten Betriebsart mit geringerer oder verschwindender zweiten Umwandlungsrate einen methanreichen Gasstrom strömen läßt.

2. Verfahren nach Anspruch 1, bei dem der methanreiche Gasstrom auf einem Zirkulationsweg (S, B) zirkuliert, der den Strömungsweg (S) wenigstens teilweise, insbesondere vollständig enthält.

3. Verfahren nach Anspruch 1 oder 2, bei dem der methanreiche Gasstrom einen Methangehalt aufweist wie das in der ersten Betriebsart erzeugte Produktgas.

4. Verfahren nach einem der vorhergehenden Ansprüche, bei dem dem Strömungsweg in der zweiten Betriebsart kein Eduktgas mehr zugeführt wird.

5. Verfahren nach einem der Ansprüche 2 bis 4, bei dem in der ersten Betriebsart das Produktgas aus einer insbesondere an dem Ende des gemeinsamen Weges von Strömungsweg oder dessen Anschlußweg und Zirkulationsweg gebildeten Weiche ausgekoppelt wird.

6. Verfahren nach einem der vorhergehenden Ansprüche, bei dem in der zweiten Betriebsart kein Produktgas mehr aus dem Reaktor ausgekoppelt wird.

7. Verfahren nach einem der Ansprüche 2 bis 6, bei dem mit dem zirkulierenden Gasstrom ein Wärmeausgleich zwischen unterschiedlichen Orten entlang des Strömungsweges und/oder Zirkulationsweges bewirkt wird.

8. Verfahren nach einem der vorhergehenden Ansprüche, bei dem die Strömungsgeschwindigkeit des auf dem Strömungsweg strömenden Gases in der zweiten Betriebsart gegenüber der ersten Betriebsart herabgesetzt ist, insbesondere auf 70 % oder weniger, bevorzugt 50% oder weniger, insbesondere 30% oder weniger.

9. Verfahren nach einem der Ansprüche 2 bis 8, bei dem der Zirkulationsweg auch in der ersten Betriebsart verwendet wird, zur anteiligen Rezirkulation des Produktgases.

10. Verfahren nach einem der vorhergehenden Ansprüche, bei dem beim Übergang von dem ersten zum zweiten Betriebszustand aufeinander abgestimmt der Eduktgasstrom heruntergefahren und der Anteil des ausgekoppelten Produktgases heruntergefahren wird, und vice versa.

11. Verfahren nach einem der vorhergehenden Ansprüche, bei dem der methanreiche Gasstrom für die Dauer bis zum erneuten Übergang zur ersten Betriebsart aufrechterhalten wird.

12. Verfahren nach einem der vorhergehenden Ansprüche, bei dem der Reaktor zwischen der ersten und der zweiten Betriebsart abhängig von der für die insbesondere raumnahe Erzeugung des Wasserstoffes für das Eduktgas bereitgestellten elektrischen Leistung intermittierend betrieben wird.

13. Verfahren nach einem der vorhergehenden Ansprüche, bei dem durchschnittlich wenigstens zehn Mal pro Monat, bevorzugt wenigstens fünf Mal pro Woche, insbesondere wenigstens zwei Mal pro Tag zwischen den Betriebsarten umgeschaltet wird.

14. Verfahren nach einem der vorhergehenden Ansprüche, bei dem die zweite Betriebsart durchschnittlich wenigstens 10 Minuten oder mehr, auch 30 Minuten oder mehr, insbesondere auch 1 Stunde oder mehr betragen kann.

15. Verfahren nach einem der vorhergehenden Ansprüche, bei dem die Schaltzeit für das Umschalten zwischen der ersten zur zweiten Betriebsart und/oder vice versa 5 Minuten oder weniger, bevorzugt 3 Minuten oder weniger, insbesondere 2 Minuten oder weniger dauert.

16. Verfahren nach einem der vorhergehenden Ansprüche, bei dem der methanreiche Gasstrom zur Vermeidung schädlicher Kohlenstoffablagerungen am Katalysator einen Anteil $H_2O$ enthält.

17. Verfahren nach Anspruch 16, bei dem der $H_2O$-Anteil überwacht und auf einen vorgegebenen Wert geregelt wird.

**Claims**

1. A method for operating a methanisation reactor (R) wherein in a first operating mode a reactant gas containing carbon dioxide and hydrogen is methanised catalytically along a flow path (S) of the reactor (R) at a first conversion rate to produce a methane-rich product gas (2),
**characterised in that**
at least at the start of the flow path in a second operating mode with a lower or insignificant second conversion rate, a methane-rich gas stream is allowed to flow.

2. The method according to Claim 1, wherein the methane-rich gas stream circulates on a circulation path (S, B) that includes at least partially, in particular entirely, the flow path (S).

3. The method according to Claim 1 or 2, wherein the methane-rich gas stream has a methane content like the product gas produced in the first operating mode.

4. The method according to any of the preceding claims, wherein reactant gas is no longer delivered to the flow path in the second operating mode.

5. The method according to any of Claims 2 to 4, wherein in the first operating mode the product gas is decoupled from a switch point formed in particular at the end of the common path of the flow path or its connection path and circulation path.

6. The method according to any of the preceding claims, wherein in the second operating mode product gas is no longer decoupled from the reactor.

7. The method according to any of Claims 2 to 6, wherein heat equalisation is brought about between different locations along the flow path and/or the circulation path by the circulating gas stream.

8. The method according to any of the preceding claims, wherein in the second operating mode the flow speed of the gas flowing on the flow path is reduced with respect to the first operating mode, in particular to 70 % or less, preferably 50 % or less, in particular 30 % or less.

9. The method according to any of Claims 2 to 8, wherein the circulation path is also used in the first operating mode in order to re-circulate the product gas proportionately.

10. The method according to any of the preceding claims, wherein upon passing from the first to the second operating state, the flow of reactant gas is shut down and the portion of decoupled product gas is shut down and vice versa, synchronized with one another.

**11.** The method according to any of the preceding claims, wherein the methane-rich gas stream is maintained for the duration until one passes back to the first operating mode.

**12.** The method according to any of the preceding claims, wherein the reactor is operated intermittently between the first and the second operating mode dependently upon the electrical power provided for the generation, in particular the spatially close generation, of the hydrogen for the reactant gas.

**13.** The method according to any of the preceding claims, wherein on average at least ten times a month, preferably at least five times a week, in particular at least two times a day, one switches between the operating modes.

**14.** The method according to any of the preceding claims, wherein the second operating mode can on average last at least 10 minutes or more, also 30 minutes or more, in particular also 1 hour or more.

**15.** The method according to any of the preceding claims, wherein the switching time for switching between the first and the second operating mode and/or vice versa is 5 minutes or less, preferably 3 minutes or less, in particular 2 minutes or less.

**16.** The method according to any of the preceding claims, wherein the methane-rich gas stream contains a portion of $H_2O$ in order to prevent harmful carbon deposits on the catalyst.

**17.** The method according to Claim 16, wherein the $H_2O$ portion is monitored and is regularised to a specified value.

**Revendications**

**1.** Procédé d'exploitation d'un réacteur de méthanisation (R), dans lequel, dans un premier mode d'exploitation, un éduit gazeux présentant du dioxyde de carbone et de l'hydrogène est méthanisé par voie catalytique en parcourant une voie d'écoulement (S) du réacteur (R) avec un premier taux de conversion pour donner un produit gazeux riche en méthane (2), **caractérisé en ce qu'** on laisse s'écouler un courant gazeux riche en méthane, au moins au début de la voie d'écoulement, dans un deuxième mode d'exploitation avec un deuxième taux de conversion moindre, voire infime.

**2.** Procédé selon la revendication 1, dans lequel le courant gazeux riche en méthane circule sur une voie de circulation (S, B) renfermant au moins partiellement, et notamment totalement, la voie d'écoulement (S).

**3.** Procédé selon la revendication 1 ou 2, dans lequel le courant gazeux riche en méthane présente une teneur en méthane égale à celle du produit gazeux issu du premier mode d'exploitation.

**4.** Procédé selon l'une des revendications précédentes, dans lequel la voie d'écoulement ne reçoit plus l'apport d'aucun éduit gazeux dans le deuxième mode d'exploitation.

**5.** Procédé selon l'une des revendications précédentes 2 à 4, dans lequel, dans le premier mode d'exploitation, le produit gazeux est extrait par un séparateur constitué, notamment à l'extrémité de la voie commune, par la voie d'écoulement ou sa voie de raccordement et par la voie de circulation.

**6.** Procédé selon l'une des revendications précédentes, dans lequel, dans le deuxième mode d'exploitation, aucun produit gazeux n'est plus extrait du réacteur.

**7.** Procédé selon l'une des revendications précédentes 2 à 6, dans lequel le courant de gaz en circulation permet de réaliser une compensation thermique entre des points différents le long de la voie d'écoulement et/ou de la voie de circulation.

**8.** Procédé selon l'une des revendications précédentes, dans lequel la vitesse d'écoulement du gaz qui s'écoule sur la voie d'écoulement subit une baisse dans le deuxième mode d'exploitation par rapport au premier mode d'exploitation, pour atteindre notamment 70 % ou moins, de préférence 50 % ou moins, notamment 30 % ou moins.

**9.** Procédé selon l'une des revendications précédentes 2 à 8, dans lequel la voie de circulation est également employée dans le premier mode d'exploitation pour une recirculation proportionnelle du produit gazeux.

**10.** Procédé selon l'une des revendications précédentes, dans lequel, lors du passage du premier au deuxième mode d'exploitation, le courant d'éduit gazeux subit une diminution graduelle et la proportion de produit gazeux extrait subit une diminution graduelle, et vice versa, lesdites diminutions étant coordonnées l'une à l'autre.

**11.** Procédé selon l'une des revendications précédentes, dans lequel le courant gazeux riche en méthane est maintenu sur la période allant jusqu'à un nouveau passage au premier mode d'exploitation.

**12.** Procédé selon l'une des revendications précédentes, dans lequel le réacteur opère de façon intermittente entre le premier et le deuxième mode d'exploitation en fonction de la puissance électrique mise à disposition pour la production, notamment locale, de l'hydrogène destiné à l'éduit gazeux.

**13.** Procédé selon l'une des revendications précédentes, dans lequel il s'effectue un changement entre les modes d'exploitation en moyenne au moins dix fois par mois, de préférence au moins cinq fois par semaine, notamment au moins deux fois par jour.

**14.** Procédé selon l'une des revendications précédentes, dans lequel le deuxième mode d'exploitation peut durer en moyenne au moins 10 minutes ou plus, voire 30 minutes ou plus, notamment 1 heure ou plus.

**15.** Procédé selon l'une des revendications précédentes, dans lequel la durée de l'opération de changement pour le passage entre le premier et le deuxième mode d'exploitation et vice versa est de 5 minutes ou moins, de préférence de 3 minutes ou moins, en étant notamment de 2 minutes ou moins.

**16.** Procédé selon l'une des revendications précédentes, dans lequel le courant gazeux riche en méthane contient une proportion de $H_2O$ afin d'éviter les dépôts carbonés nocifs dans le catalyseur.

**17.** Procédé selon la revendication 16, dans lequel la proportion de $H_2O$ est surveillée et régulée à une valeur prédéfinie.

Fig. 1

Fig. 2